# EUROPEAN PATENT APPLICATION

(11) **EP 3 771 477 A1**
(43) Date of publication of application: **03.02.2021**
(21) Application number: 19189585.3
(22) Date of filing: 01.08.2019
(51) Int. Cl.: A61M 39/10, A61M 25/00, F16L 19/02, F16L 27/08, F16L 37/088, F16L 37/53, A61M 39/12

(54) **CONNECTION DESIGN FOR A TAVI HANDLE LUER LOCK**

(71) Applicant: BIOTRONIK AG, 8180 Bülach (CH)
(72) Inventor: Fargahi, Amir, 8180 Buelach (CH)
(74) Representative: Randoll, Sören

(57) **Abstract**

The present invention relates to a connection system (1) for connecting a first component (10) of a medical device (2) to a second component (50, 5) of the medical device (2), comprising: a first component (10) of the medical device (2), wherein the first component (10) comprises a connecting portion (11), wherein the connecting portion (11) forms a stop surface (14), a second component (50, 5) of the medical device (2), an annular sealing member (40) configured to be arranged on the second component (50, 5), a retaining member (30) configured to be arranged on the second component (50, 5) such that the retaining member (30) is fastened to the second component (50, 5), a lid member (20) configured to receive the second component (50, 5), wherein the lid member (20) is configured to be connected to the connecting portion (11) such that the lid member (20) presses against the retaining member (30) and a face side (52) of the second component (50, 5) contacts the stop surface (14), wherein the sealing member (40) is arranged between the stop surface (14) and the retaining member (30) to seal a flow path formed by said first and second components (10, 50, 5).

## Description

The present invention relates to a connection system for connecting a first component of a medical device to a second component of the medical device. Particularly, the medical device can be a catheter, e.g. a balloon catheter, particularly a catheter for a transcatheter aortic valve implantation (TAVI).

Usually such connections are based on adhesives, e.g. a UV curable adhesive.

Particularly, EP 2 585 165 B1 discloses an accommodating connector configured to improve fluid flow and reduce blood clotting in a fluid flow between a medical device and a source of fluid, wherein the accommodating connector comprises a spring-loaded self-adjusting tube that protrudes from the accommodating connector and is part of the fluid path and a sealing mass partially or fully surrounding the tube.

Furthermore, US 6,193,692 B1 discloses a sheath that is connected via a cylindrical body and a seal to a stylet subassembly using a Luer lock.

Further, US 9,126,029 B2 discloses a medical fluid connector configured to inhibit disconnection, the connector comprising an o-ring contacting both a portion of a distally extending plunger and a portion of a proximally extending wall in a region where the distally extending plunger and the proximally extending wall overlap.

Furthermore, US 9,259,559 B2 discloses a hub of a catheter, wherein an outer tubular member of a catheter shaft is inserted into the distal end of the hub and secured to an inside surface of the hub with an adhesive to provide a fluid tight seal.

However, processes involving connections based on adhesives are complex and difficult to automate.

Thus, based on the above, the problem to be solved by the present invention is to provide a simple and safe design that allows connecting the two components of a medical device in a fast, safe and easy to automate process.

This problem is solved by a connection system having the features of claim 1. Preferred embodiments are stated in the corresponding sub claims and are described below.

According to claim 1, a connection system is disclosed, comprising:
- a first component of the medical device, wherein the first component comprises a connecting portion, wherein the connecting portion forms a stop surface,
- a second component of the medical device,
- an annular sealing member (e.g. an O-ring) configured to be arranged on the second component,
- a retaining member configured to be arranged on the second component such that the retaining member is fastened to the second component,
- a lid member configured to receive the second component, wherein the lid member is configured to be connected to the connecting portion such that the lid member presses against the retaining member and a face side of the second component contacts the stop surface, wherein the sealing member is arranged between the stop surface and the retaining member to seal a flow path between said first component and said second component.

According to an embodiment of the connection system, the lid member is configured to be connected to the connecting portion through one of: a latching connection, a screw joint, a weld seam.

Furthermore, according to an embodiment of the connection system, the retaining member comprises an annular portion comprising an inner edge, and wherein the retaining member comprises a plurality of protrusions, wherein each protrusion protrudes radially inwards from the inner edge of the annular portion, particularly in an inclined fashion with respect to an axial direction of the annular portion.

Furthermore, according to an embodiment of the connection system, the annular portion comprises a gap. Particularly, this gap allows to arrange the retaining member on the second component by pushing the second component through the gap so that the annular portion encompasses the second component.

Furthermore, according to an embodiment of the connection system, the respective protrusion of the retaining member is configured to press against the second component or to engage with a groove formed in the second component to fasten the retaining member to the second component when the retaining ring is arranged on the second component.

Furthermore, according to an embodiment of the connection system, the medical device is a catheter. According to an embodiment, the catheter can be a TAVI catheter. Further, the catheter can be a balloon catheter.

Furthermore, according to an embodiment of the connection system, the first component is a connector, particularly a connector of a catheter, particularly a Luer lock connector.

Furthermore, according to an embodiment of the connection system, the second component is a sheath (e.g. of a catheter), particularly a metallic sheath.

Furthermore, according to an alternative embodiment of the connection system, the first component is a catheter hub comprising a first port for introducing a fluid medium (e.g. an inflation medium for inflating a balloon of the catheter) and a second port for insertion of a guide wire. Here, according to an embodiment of the connection system, the second component is a sheath of the catheter, wherein in an embodiment this sheath is connected to a balloon of the catheter and comprises an inflation lumen configured to pass the fluid medium into an interior space of the balloon.

Furthermore, according to an embodiment, the connection system is further configured to connect a third component of the medical device (e.g. catheter) to the first component.

Particularly, the third component is an inner sheath of the catheter/medical device comprising a guide wire lumen for receiving a guide wire, wherein particularly said sheath connected to the balloon surrounds a section of the guide wire lumen and/or of the inner sheath.

Furthermore, according to an embodiment of the connection system, for connecting the third component to the first component, the connection system comprises: a further annular sealing member (e.g. an O-ring) configured to be arranged on the third component, a further retaining member configured to be arranged on the third component such that the further retaining member is fastened to the third component, and a further lid member configured to receive the third component, wherein the further lid member forms a stop surface and is configured to be connected to a further connecting portion of the first component such that a face side of the third component contacts the stop surface of the further lid member and the further sealing member is arranged between the stop surface of the further lid member and the further retaining member. Particularly, the further retaining member can be designed in the same way as the retaining member described above.

According to a further aspect of the present invention, a medical device is disclosed, wherein the medical device comprises a connection system according to the present invention. Particularly, the medical device can be one of: a catheter, a balloon catheter, a TAVI catheter.

According to yet another aspect of the present invention, a method for connecting a first component of a medical device to a second component of the medical device is disclosed, wherein the method uses a connection system according to the present invention, and wherein the method comprises the steps of:
- arranging the retaining member on the second component such that the retaining member is fastened to the second component,
- arranging the annular sealing member on the second component, and
- connecting the lid member to the connecting portion before or after said arranging of the retaining member and of the annular sealing member.

In the following, embodiments of the present invention as well as further aspects, features and advantages of the present invention are described with reference to the Figures, wherein
- Fig. 1: shows a schematically exploded view of an embodiment of a connection system according to the present invention that is configured to provide a connection between a first component and a second component of a medical device (e.g. catheter);
- Figs. 2A-2B: show fastening of a lid member of an embodiment of the connection system to a connecting portion of the first component of the medical device;
- Figs. 3A-3B: show embodiments of retaining members of a connection system according to the present invention;
- Fig. 4: shows a perspective view of a retaining member arranged on the second portion of the medical device;
- Fig. 5: shows a part of a catheter comprising a first component in form of a Luer lock connector and a second portion in form of a metallic sheath;
- Fig. 6: shows a cross sectional view of the first component shown in Fig. 5;
- Fig. 7: shows a schematical exploded view of a connection system according to the present invention that is configured to connect a connector of a catheter to a sheath of the catheter;
- Fig. 8: shows a schematical cross sectional view of the connection system shown in Fig. 7 in a state where the sheath is connected to the connector;
- Fig. 9: shows a schematical cross sectional view of an alternative way to fasten a lid member of the connection system to the connector using a screw joint;
- Fig. 10: shows a schematical cross sectional view of a further alternative way to fasten the lid member of the connection system to the connector using a weld seam;
- Fig. 11: shows a schematical exploded view of a further embodiment of the connection system applied to a catheter hub;
- Fig. 12: shows a schematical cross sectional view of the hub shown in Fig. 11, wherein a sheath and an inner sheath are connected to the hub using the connection system; and
- Figs. 13-15: shows a process of connecting the sheaths of the catheter to the hub shown in Figs. 11 and 12 with help of the connection system according to the present invention.

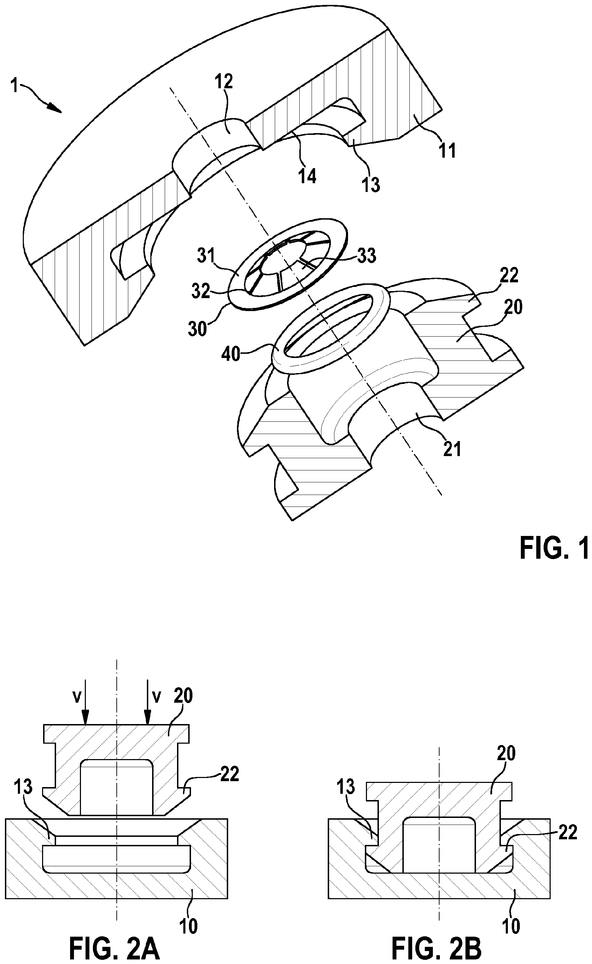

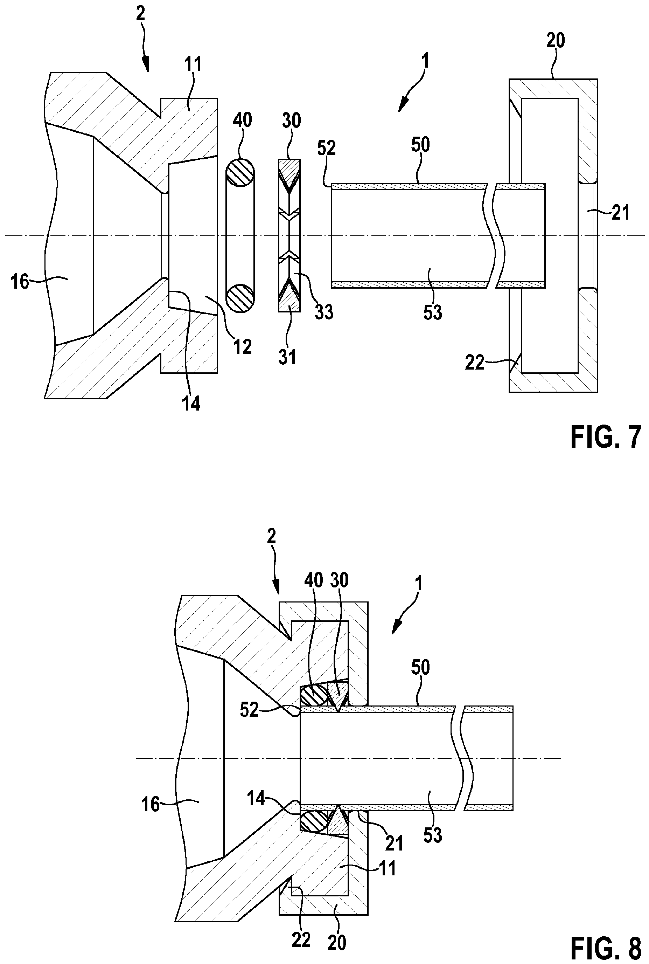

Fig. 1 shows an embodiment of a connection system 1 according to the present invention for connecting a first component 10 of a medical device 2 to a second component 50 of the medical device 2 (cf. also Figs. 7 and 8).

This concept of providing a connection between the two components (e.g. connector 10 and sheath 50 of a catheter 2) of a medical device 2 merely requires a sealing member 40 such as an O-ring to create a seal between the first component 10 and the second component 50, wherein an (e.g. push-on) retaining member (such as an retaining ring) 30 creates the necessary force between the second component 50 and the first component 10. A lid member 20 realizes the position of the second component 50 (cf. Figs 7 and 8), which is positioned up to a stop surface 14 of the connecting portion 11 of the first component 10. Particularly, the lid member 20 is fixed to the first portion 10, particularly to the connecting portion 11, using a latching connection (cf. Fig. 1, 2A, 2B and Figs. 7 and 8). The sealing member 40 and the retaining member 30 remain fixed between the connecting portion 11/stop surface 14 and the lid member 20.

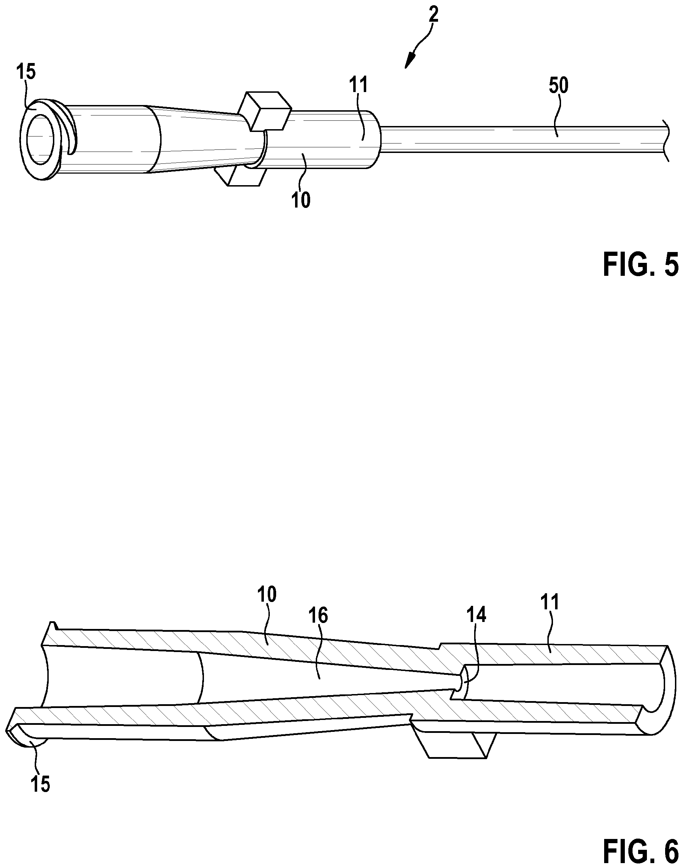

Particularly, as indicated in Figs. 5 and 6, the medical device 2 can be a catheter 2, wherein the first component 10 can be a connector 10, such as a Luer lock connector 10 that comprises an outer thread 15 at a proximal end of the connector 10. As further shown in Fig. 5, the second component can be a sheath 50, particularly a metallic sheath 50, of the catheter 2. The connector 10 provides a path 16 to a lumen of the sheath 50 and can be formed out of a polyamide, a polycarbonate, or ABS. Furthermore, the sealing member 40 can be formed out of a silicone with various Shore hardnesses, or polyurethane (PUR). Furthermore, the retaining member 30 can be formed out of a steel, particularly a stainless steel.

As indicated e.g. in Figs. 7 and 8, the lid member 20 is configured to be connected to the connecting portion 11 through a latching connection according to an embodiment. For this, the lid member 20 may comprise a protrusion 22 configured to engage behind the connecting portion 11 of the first component 10 so as to fasten the lid member 20 to the connecting portion 11. Alternatively, as shown in Figs. 1 and 2A, 2B, the lid member 20 may be configured to engage behind an internal protrusion 13 of the connecting portion 11 with a protrusion 22 of the lid member 20, wherein said protrusion 22 of the lid member extends radially outwards.

Both the lid member 20 and the connecting portion 11 comprise an opening 21, 12 for receiving the second portion / sheath 50, wherein the sealing member 40 and the retaining member are arranged between the lid member 20 and the stop surface 14 of the connecting portion 11 (cf. Figs. 1 and 8).

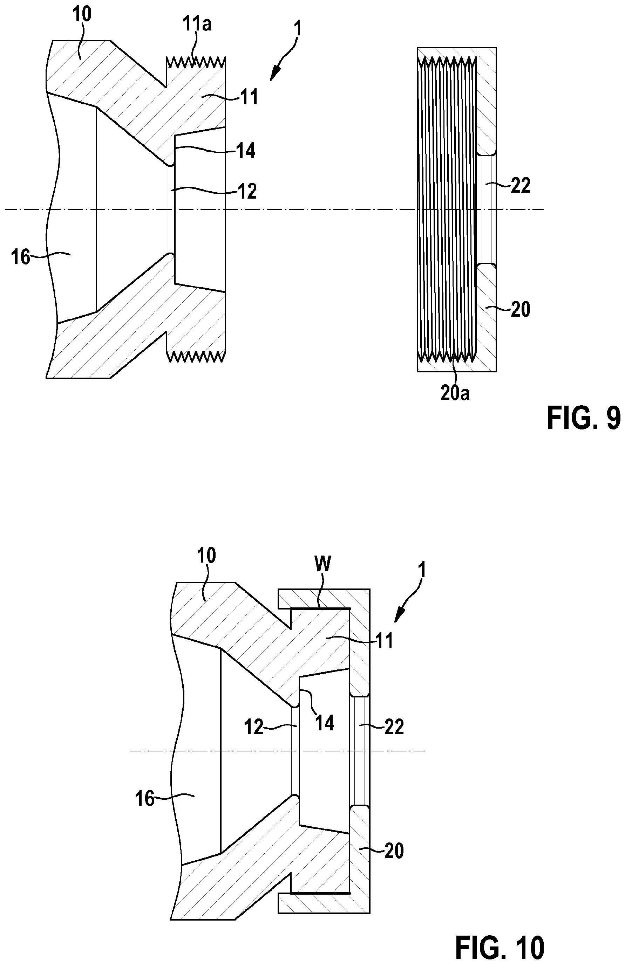

Alternatively, instead of a latching connection between the lid member 20 and the connecting portion 11 of the first component 10, a screw joint can be used to fasten the lid member 20 to the connecting portion 11, which is shown in Fig. 9. Here, the lid member 20 comprises an inner thread 20a that is configured to engage with an outer thread 11a provided on the connecting portion 11 of the first component 10.

According to a further alternative embodiment shown in Fig. 10, the latching connection 22, 11 or screw joint 20, 11a between the lid member 20 and the connecting portion 11 can also be replaced by a weld seam W formed between the lid member 20 and the connecting portion 11 of the first component 10 (e.g. by means of ultrasound welding).

In order to mechanically fasten the retaining member 30 to an outside of 50a of the second portion 50 (particularly sheath 50 of catheter 2), the retaining member 30 can comprise an annular portion 31 comprising an inner edge 32, as well as a plurality of elastically bendable protrusions 33, wherein each protrusion 33 protrudes radially inwards from the inner edge 32 of the annular portion 31, particularly in an inclined fashion, as shown in Figs. 1, 7 and 8. Thus, when the retaining member 30 is pushed on the second component 50 in a first direction (the protrusions are then inclined in an opposite second direction) pointing towards a distal end of the second component 50 so that its annular portion 31 surrounds the second portion 50, the respective inclined protrusion 33 presses against the surface 50a of the second component 50 so that the retaining member 30 resists a movement with respect to the second component 50 in the second direction. This allows to push the face side 52 of the second component 50 against the stop surface 14 of the connecting portion 11 by means of the lid member 20 pressing against the retaining member 30 in the second direction (cf. e.g. Fig. 8).

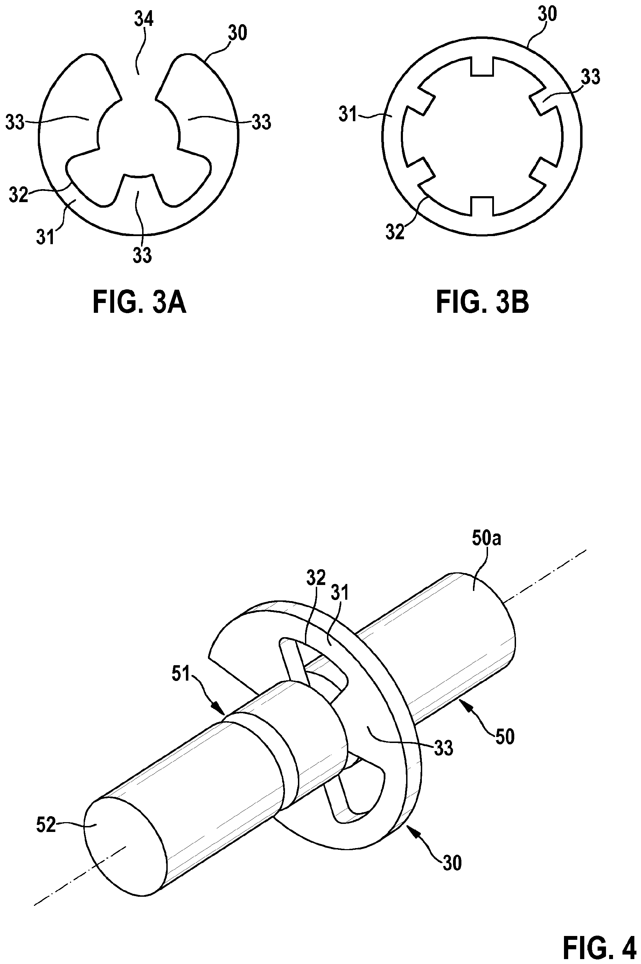

As shown in Figs. 3A the retaining member 30 can also comprise a gap 34 in its annular portion 31 to place the retaining member 30 with its protrusions 33 in a groove 51 of the second portion / sheath 50 as shown in Fig. 4. Here, the protrusions 33 do not need to assume an inclined shape as described above.

According to Fig. 3B, the retaining member 30 configured to be received in said groove 51 (cf. Fig. 4) can also comprise a closed annular portion 31.

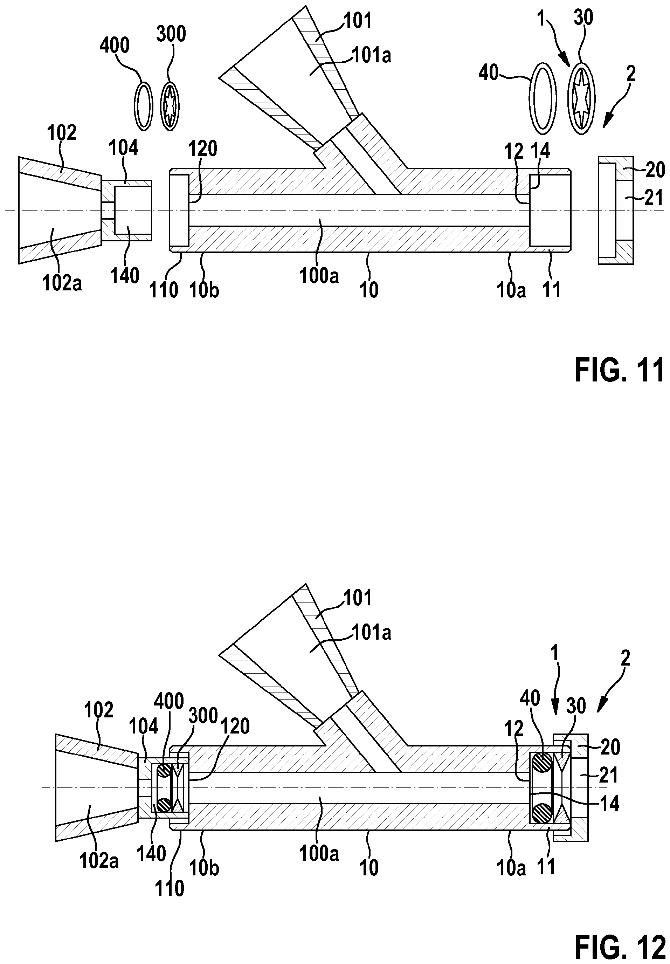

According to yet another embodiment shown in Fig. 11 to 15, the connection system 1 according to the present invention can also be used to connect an outer sheath 5 to a first component 10 of the catheter 2 in the form of a hub 10. The outer sheath 5 can be connected to a balloon 6 at a distal end of the sheath 5 and can comprise an inflation lumen for inflating the balloon 6.

Particularly, the catheter hub 10 comprises a first port 101 providing a flow path 101a for introducing a fluid medium into the hub 10 and into the inflation lumen of the sheath 5 and a second port 102 providing a path for inserting a guidewire into a guidewire lumen 4a of an inner shaft 4 of the catheter 2.

Here, the lid member 20 comprises an opening 21 for receiving sheath 5, wherein the sealing member 40 and the retaining member 30 are configured to be arranged on the sheath 5. As before, the retaining member 30 is thereby fastened to the sheath 5 and allows pressing a face side 52 of the sheath 5 against a stop surface 14 of a connecting portion 11 of the hub 10, which connecting portion 11 is provided on a distal end portion 10a of the hub 10. The retaining member 30 can be formed as described above in conjunction with Figs. 1, 3A, 3B, and 4. Again, the sealing member 40 is arranged between the stop surface 14 and the retaining member 30 and seals a flow path between the sheath 5 and a lumen 100a of the hub 10. The lid member 20 can be connected to the connecting portion 11 as described above (e.g. by means of a latching connection, a screw joint, or a weld seam). Thus, an inflation medium can be passed through flow path 101a of the first port 101 and lumen 100a into said inflation lumen of the sheath 5.

Furthermore, the connection system 1 can also be used to connect a third component 4 in form of an internal sheath 4 of the catheter 2 to the hub 10 at a proximal end portion 10b of the hub 10 at which a further connecting portion 110 is provided. The further connecting portion 110 also comprises an opening 120 for receiving the internal sheath 4 as will be described further below.

Particularly, as indicated in Fig. 11, the internal sheath 4 extends out of the sheath 5 at a proximal end of the sheath 5 and comprises a guidewire lumen 4a for receiving a guidewire of the catheter 2.

Further, the second port 102 of the hub 10 is preferably provided on a further lid member 104 that is configured to be connected to the further connecting portion 110 of hub 10 (e.g. as described above by means of a latching connection, a screw joint, or a weld seam).

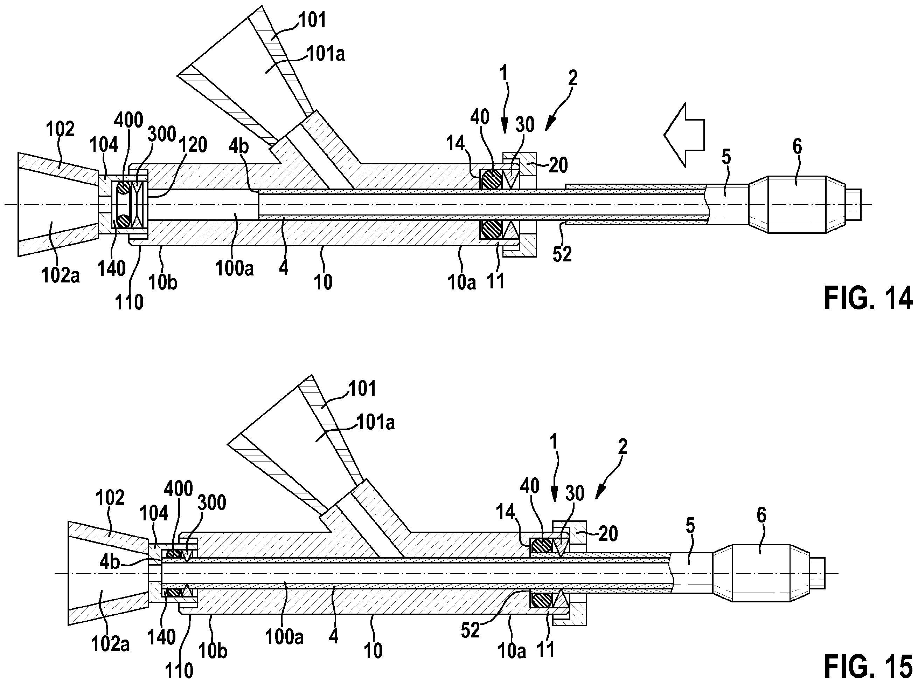

For connecting the internal sheath 4 to the hub 10 at the proximal end portion 10b, a further retaining member 300 and a further sealing member 400 are provided, wherein the further retaining member 300 can be designed as described above in conjunction with Figs. 1, 3A, 3B and 4. The further retaining member 300 is placed on the internal sheath 4 and the further sealing member 400 is arranged between a stop surface 140 of the further lid member 104 and the further retaining member 300. As shown in Fig. 15, the further sealing member 400 seals a path comprising the path 102a formed by the second port 102 and the guidewire lumen 4a. Particularly, the retaining member 300 is placed such on the internal sheath 4 that the face side 4b of the internal sheath 4 contacts the stop surface 140 of the further lid member 104.

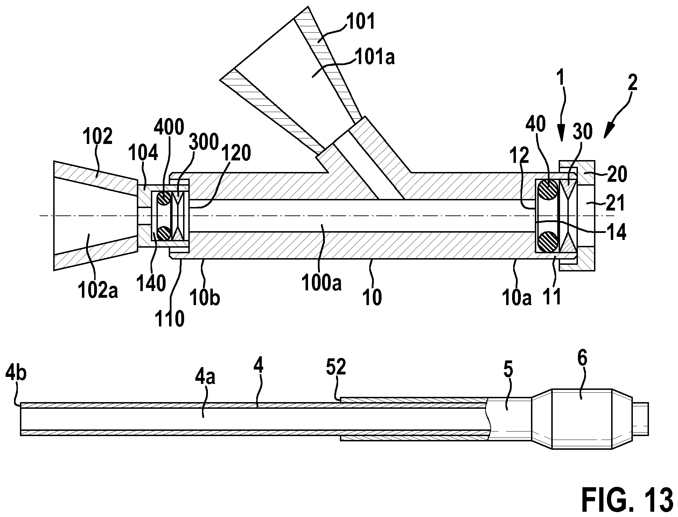

As shown in Figs. 13 to 15, the sheath 5 and internal sheath 4 can be connected to the hub 10, by pre-mounting the lid members 20, 104 to the connecting portions 11, 110 of the hub 10 as detailed in Fig. 13, wherein the sealing members 40, 400 and retaining members 30, 300 are placed in the respective chamber formed by the respective lid member 20, 104 and the associated connecting portion 11, 110. Then the internal sheath 4 is inserted with its face side 4b ahead into the opening 21 of the lid member 20 and through the retaining member 30 and sealing member 40 into the lumen 100a of the hub 10 (via opening 12) and further via said opening 120 through the further retaining member 300 and the further sealing member 400 until the face side 4b butts against the stop surface 140 of the further lid member 104. At this point, also the face side 52 of the sheath 5 butts against the stop surface 14 of lid member 20. Due to the retaining members 30, 300 a movement of the sheaths 4, 5 in the opposite direction is prevented.

The present invention offers the advantage that the connector (e.g. Luer lock connector) can be manufactured externally ready to use (with a sealing member, retaining ring and lid member). Furthermore, the connection made with help of the connection system needs no curing time in comparison to gluing processes (UV adhesive) and also involves no downtime as with 2-component adhesives. Furthermore, a secure connection is achieved that does not require wetting of surfaces compared to usual bonding processes. Since gluing processes are sensitive, the solution according to the present invention has the benefit of avoiding corresponding risks related to gluing processes. Furthermore, the mechanical connection according to the present invention is relatively easy to automate.

In view of all the foregoing disclosure, further embodiments of the present invention are reflected by the following consecutively numbered embodiments:
1. Connection system (1) for connecting a first component (10) of a medical device (2) to a second component (50, 5) of the medical device (2), comprising:
   - a first component (10) of the medical device (2), wherein the first component (10) comprises a connecting portion (11), wherein the connecting portion (11) forms a stop surface (14),
   - a second component (50, 5) of the medical device (2),
   - an annular sealing member (40) configured to be arranged on the second component (50, 5),
   - a retaining member (30) configured to be arranged on the second component (50, 5) such that the retaining member (30) is fastened to the second component (50, 5),
   - a lid member (20) configured to receive the second component (50, 5), wherein the lid member (20) is configured to be connected to the connecting portion (11) such that the lid member (20) presses against the retaining member (30) and a face side (52) of the second component (50, 5) contacts the stop surface (14), wherein the sealing member (40) is arranged between the stop surface (14) and the retaining member (30) to seal a flow path formed by said first and second components (10, 50, 5).
2. The connection system according to embodiment 1, **wherein** the lid member (20) is configured to be connected to the connecting portion (11) through one of: a latching connection, a screw joint, a weld seam (W).
3. The connection system according to embodiment 1 or 2, **wherein** the retaining member (30) comprises an annular portion (31) comprising an inner edge (32), and wherein the retaining member (30) comprises a plurality of protrusions (33), wherein each protrusion (33) protrudes radially inwards from the inner edge (32) of the annular portion (31).
4. The connection system according to embodiment 3, **wherein** the annular portion (31) comprises a gap (34).
5. The connection system according to embodiment 3 or 4, **wherein** the respective protrusion (33) is configured to press against the second component (50, 5) or to engage with a groove (51) formed in the second component (50) to fasten the retaining member (30) to the second component (50) when the retaining member (30) is arranged on the second component (50).
6. The connection system according to one of the preceding embodiments, **wherein** the medical device is a catheter (2).
7. The connection system according to one of the preceding embodiments, **wherein** the first component is a connector (10), particularly a Luer lock connector.
8. The connection system according to one of the preceding embodiments, **wherein** the second component is a sheath (50), particularly a metallic sheath.
9. The connection system according to one of the embodiments 1 to 6, **wherein** the first component is a catheter hub (10) comprising a first port (101) for introducing a fluid medium and a second port (102) for receiving a guidewire.
10. The connection system according to one of the embodiments 1 to 6 or according to embodiment 9, **wherein** the second component is a sheath (5).
11. The connection system according to embodiment 9 or 10, **wherein** the connection system (1) is further configured to connect a third component (4) of the medical device (2) to the first component (10).
12. The connection system according to embodiment 11, **wherein** the third component is an inner sheath (4) comprising a guide wire lumen (4a) for receiving a guidewire.
13. The connection system according to embodiment 11 or 12, **wherein** the connection system comprises: a further annular sealing member (400) configured to be arranged on the third component (4), a further retaining member (300) configured to be arranged on the third component (4) such that the further retaining member (300) is fastened to the third component (4), and a further lid member (104) configured to receive the third component (4), wherein the further lid member (104) forms a stop surface (140) and is configured to be connected to a further connecting portion (110) of the first component (10) such that a face side (4b) of the third component (4) contacts the stop surface (140) of the further lid member (104) and the further annular sealing member (400) is arranged between the stop surface (140) of the further lid member (104) and the further retaining member (300).
14. A medical device (2) comprising a connection system (1) according to one of the preceding embodiments.
15. A method for connecting a first component (10) of a medical device (2) to a second component (50, 5) of the medical device (2), wherein the method uses a connection system (1) according to one of the preceding embodiments, wherein the method comprises the steps of:
   - arranging the retaining member (30) on the second component (50, 5) such that the retaining member (30) is fastened to the second component (50, 5),
   - arranging the annular sealing member (40) on the second component (50, 5), and
   - connecting the lid member (20) to the connecting portion (11) before or after said arranging of the retaining member (30) and of the annular sealing member (40).

## Claims

1. Connection system (1) for connecting a first component (10) of a medical device (2) to a second component (50, 5) of the medical device (2), comprising:
- a first component (10) of the medical device (2), wherein the first component (10) comprises a connecting portion (11), wherein the connecting portion (11) forms a stop surface (14),
- a second component (50, 5) of the medical device (2),
- an annular sealing member (40) configured to be arranged on the second component (50, 5),
- a retaining member (30) configured to be arranged on the second component (50, 5) such that the retaining member (30) is fastened to the second component (50, 5),
- a lid member (20) configured to receive the second component (50, 5), wherein the lid member (20) is configured to be connected to the connecting portion (11) such that the lid member (20) presses against the retaining member (30) and a face side (52) of the second component (50, 5) contacts the stop surface (14), wherein the sealing member (40) is arranged between the stop surface (14) and the retaining member (30) to seal a flow path formed by said first and second components (10, 50, 5).

2. The connection system according to claim 1, **wherein** the lid member (20) is configured to be connected to the connecting portion (11) through one of: a latching connection, a screw joint, a weld seam (W).

3. The connection system according to claim 1 or 2, **wherein** the retaining member (30) comprises an annular portion (31) comprising an inner edge (32), and wherein the retaining member (30) comprises a plurality of protrusions (33), wherein each protrusion (33) protrudes radially inwards from the inner edge (32) of the annular portion (31).

4. The connection system according to claim 3, **wherein** the annular portion (31) comprises a gap (34).

5. The connection system according to claim 3 or 4, **wherein** the respective protrusion (33) is configured to press against the second component (50, 5) or to engage with a groove (51) formed in the second component (50) to fasten the retaining member (30) to the second component (50) when the retaining member (30) is arranged on the second component (50).

6. The connection system according to one of the preceding claims, **wherein** the medical device is a catheter (2).

7. The connection system according to one of the preceding claims, **wherein** the first component is a connector (10), particularly a Luer lock connector.

8. The connection system according to one of the preceding claims, **wherein** the second component is a sheath (50), particularly a metallic sheath.

9. The connection system according to one of the claims 1 to 6, **wherein** the first component is a catheter hub (10) comprising a first port (101) for introducing a fluid medium and a second port (102) for receiving a guidewire.

10. The connection system according to one of the claims 1 to 6 or according to claim 9, **wherein** the second component is a sheath (5).

11. The connection system according to claim 9 or 10, **wherein** the connection system (1) is further configured to connect a third component (4) of the medical device (2) to the first component (10).

12. The connection system according to claim 11, **wherein** the third component is an inner sheath (4) comprising a guide wire lumen (4a) for receiving a guidewire.

13. The connection system according to claim 11 or 12, **wherein** the connection system comprises: a further annular sealing member (400) configured to be arranged on the third component (4), a further retaining member (300) configured to be arranged on the third component (4) such that the further retaining member (300) is fastened to the third component (4), and a further lid member (104) configured to receive the third component (4), wherein the further lid member (104) forms a stop surface (140) and is configured to be connected to a further connecting portion (110) of the first component (10) such that a face side (4b) of the third component (4) contacts the stop surface (140) of the further lid member (104) and the further annular sealing member (400) is arranged between the stop surface (140) of the further lid member (104) and the further retaining member (300).

14. A medical device (2) comprising a connection system (1) according to one of the preceding claims.

15. A method for connecting a first component (10) of a medical device (2) to a second component (50, 5) of the medical device (2), wherein the method uses a connection system (1) according to one of the preceding claims, wherein the method comprises the steps of:
- arranging the retaining member (30) on the second component (50, 5) such that the retaining member (30) is fastened to the second component (50, 5),
- arranging the annular sealing member (40) on the second component (50, 5), and
- connecting the lid member (20) to the connecting portion (11) before or after said arranging of the retaining member (30) and of the annular sealing member (40).
